# EUROPEAN PATENT APPLICATION

(11) **EP 3 431 168 A1**
(43) Date of publication of application: **23.01.2019**
(21) Application number: 17182039.2
(22) Date of filing: 19.07.2017
(51) Int. Cl.: B01D 61/14, B01D 61/24, B01D 61/28, B01D 63/02, A61K 47/68

(54) **ÉLIMINATION DE MÉDICAMENT NON LIÉ APRÈS COUPLAGE CONJUGUÉ ANTICORPS-MÉDICAMENT**

(71) Applicant: Bayer Aktiengesellschaft, 51373 Leverkusen (DE)
(72) Inventor: Schwan, Peter, 51373 Leverkusen (DE)
(74) Representative: BIP Patents

(57) **Abstract**

Disclosed herein is a unit for ultrafiltration and purification (1) of a product stream (3) containing a biological macromolecular product as well as a substance to be separated from the product stream, comprising at least one capillary ultrafiltration module (2), characterized in that
- at least one pump (4) conveys the product stream into the capillaries of the at least one capillary ultrafiltration module,
- at least one positive displacement pump (5) conveys the product stream from the capillaries and
- at least one further pump (6) passes a washing fluid (9) over the outside of the capillaries,
- the unit includes no measure for circulating the product stream and the washing fluid into the ultrafiltration module, as well as
at least one guard purifier (8), through which all fluid leaving the at least one capillary ultrafiltration module in the direction of the product stream is passed.

## Description

The fact that antibodies can selectively recognize very specific structures in the body, with which they interact and bond, makes them attractive for use in transporting active substances directly to the precise focus of a disease.

Antibody drug conjugates (ADCs) comprise antibodies covalently attached to highly potent drugs. For the attachment a variety of conjugation technologies can be used. As therapeutics ADCs combine the exquisite specificity of antibodies, enabling discrimination between healthy and diseased tissue, with the cell-killing ability of drugs, e.g. cytotoxic drugs. In other words, the antibody is for example used to transport a cytotoxic substance directly to a cancer cell and release it inside the cell. In this way the healthy cells are largely spared the toxic side-effects that occur with traditional chemotherapy.

In detail, ADCs consist of three different components: the antibody, a drug e.g. a cytotoxic active ingredient (the toxophore), and a so-called 'linker' which connects the antibody and the toxophore. The antibody recognizes and binds to certain protein molecules ('tumor markers') on the surface of tumor cells. This binding process triggers a mechanism which transports the ADC inside the cell, where the drug (e.g. the toxophore) is separated from the antibody and can act on the cancer cell for example via blocking important cell functions are thereby leading to programmed cell death (apoptosis). Thus, it is essential for the success of a therapy with ADCs that the conjugate does not lose its conjugated drug as its passes through the body, and does not release it until it is inside the cancer cell. The 'linker' is therefore designed in such a way that it can only be split inside cancer cells.

However, during the process of coupling the antibodies to the drugs a mixture of correctly conjugated antibody drug conjugates as well as unbound drug is always generated. Hence, prior to using the ADCs this solution containing antibody-drug-conjugates as well as unbound drug needs to be purified under stringent conditions to minimize the content of unbound drug.

This stringent purification is achieved via batchwise diafiltration of the solution containing antibody-drug-conjugates as well as unbound drugs using high diafiltration factors.

ADC complexes generally need to be handled with care in order to prevent damage of the complex such as fragmentation and/or aggregate formation e.g. formation of subvisible particles upon storage. Hence shorter processing times can be beneficial for the stability of ADC complexes. These can be achieved via larger membrane areas. However, this measure would lead to higher pump rates and potentially to lower yields. In addition, a scale-up could on the one hand can carry the risk that due to an insufficient mixing an inadequate separation of the product from the unbound drug takes place and on the other hand that a higher mixing rate, which avoids the previously mentioned draw-back results in damage of the ADC complex.

In order to realize more flexible production processes this step of separation of unbound drug from the ADCs should ideally be reliably and robust under various process conditions as well as easy to scale up.

Therefore, it was an object of the present invention to provide more flexible production processes in which the step of separation of unbound drug from the ADCs should ideally be reliably and robust under various process conditions as well as easy to scale up.

This object is achieved via providing a unit for ultrafiltration and purification (1) of a product stream (3) containing a biological macromolecular product as well as a substance to be separated from the product stream, comprising at least one capillary ultrafiltration module (2), characterized in that
- at least one pump (4) conveys the product stream into the capillaries of the at least one capillary ultrafiltration module,
- at least one positive displacement pump (5) conveys the product stream from the capillaries and
- at least one further pump (6) passes a washing fluid (9) over the outside of the capillaries,
- the unit includes no measure for circulating the product stream and the washing fluid into the ultrafiltration module, as well as
- at least one guard purifier (8), through which all fluid leaving the at least one capillary ultrafiltration module in the direction of the product stream is passed.

Said unit has the effect that it reliably ensures a separation of the substance to be separated from the product stream under low shear forces thus avoiding the risk of formation of subvisible particles and aggregate formation upon long-time storage. This is the case as usually already the at least one capillary ultrafiltration module with the features as described above will reliably remove all substance to be separated from the product stream. In addition the guard purifier acts as safeguard to ensure that the concentration of the substance to be separated from the product stream is below a given threshold once the retentate leaves the at least one guard purifier.

In other words in the case of a product stream comprising antibody drug conjugates as biological macromolecular products as well as toxophores as substance to be separated from the product stream the unit for ultrafiltration and purification as described herein reliably ensures a separation of the unbound toxophores from the antibody drug conjugates in the product stream while avoiding the risk of formation of subvisible particles and aggregate formation upon long-time storage.

This effect is caused by the combination of one or more ultrafiltration modules equipped as detailed above and at least one guard purifier, which serves as safeguard for any remaining unbound drug particles.

As used herein the term "guard purifier" refers to a device which is suited to remove the substance to be separated from the product stream, e.g. via binding to it.

As used herein the term "antibody-drug-conjugate (ADC)" refers to a complex comprising at least one antibody, at least one drug and at least one 'linker' which connects the antibody with the drug.

As used herein, the terms "drug," "therapeutic," or "active agent" refer to any molecule, molecular complex or substance administered to an organism for diagnostic or therapeutic purposes, including the treatment of a disease or infection, medical imaging, monitoring, contraceptive, cosmetic, nutraceutical, pharmaceutical and prophylactic applications. The term "drug" includes any such molecule, molecular complex or substance that is chemically modified and/or operatively attached to a biologic or biocompatible structure. The term "prodrug" refers to a drug, drug precursor or modified drug that is not fully active or available until converted in vivo to its therapeutically active or available form.

As used herein the term "substance to be separated from the product stream" refers to a substance, from which the final product is to be purified e.g. because said substance has detrimental effects on organism or the environment. However, there can be other reasons for which the final product is to be purified from "substance to be separated from the product stream".

As used herein the term "product stream" refers to the fluid comprising the biopharmaceutical biological macromolecular products passing the unit for ultrafiltration and purification described herein. In other words, prior to entering the unit for ultrafiltration and purification the product stream can also be referred to as feed and upon leaving the unit for ultrafiltration and purification the product stream can also be referred to as retentate.

One example of a "substance to be separated from the product stream" is a toxophore.

The term "toxophore" as used herein refers to a chemical group that produces a toxic effect when administered to a cell.

As used herein the term "unit" or "unit operation" refers to a device or a combination of devices that perform one process step in a production process of a biological macromolecular product. In other words, in order to provide the final a biological macromolecular product several units will have to be passed by the product stream.

As used herein the term "subvisible particles" refers to proteinaceous and non-proteinaceous particles with a size between 0,1 µm - 100 µm.

As used herein the term "ultrafiltration" refers to a process that involves removal or separation of components (permeable molecules like salts, small proteins, solvents etc.,) of a solution based on their molecular size regardless of whether the concentration of individual components - e.g. a product - is altered or not. For this process filters permeable for molecules between 1 and 100000 Da are used. Forces e.g. pressure are applied to drive the ultrafiltration process.

Preferably filters permeable for molecules between 1 and 1000 Da, more preferably permeable for molecules between 1 and 10.000 Da, most preferable permeable for molecules between 1 and 30.000 Dare used.

As used herein the term "diaflltration" refers to a specific type of ultrafiltration in which the buffer of a suspension is exchanged or the salt concentration is altered, but the concentration of a product contained in the suspension is more or less maintained. If a batchwise processing is used for diafiltration solvents are constantly added to the retentate until the previous solvent is replaced via the filtration membrane. This solvent replacement i.e. the overall mass transfer is driven by a combination of diffusion and a separate force or separate forces e.g. pressure.

As used herein the term "dialysis" refers to the process of separating molecules in solution exclusively by the difference in their rates of diffusion, without applying any other forces such as pressure. However, if the difference in concentration of the molecules to be separated on the two sides of the filtration membrane becomes too large a net flow can occur from one side of the filtration membrane to the other. The forces generated by said net flow can cause molecules to pass the filtration membrane, which should be retained due to their molecular weight. Hence, this net flow can cause undesired molecules to be present in the retentate or can cause the loss of product, if product is transferred to the permeate. Moreover, the net flow negatively influences the rate at which molecules are exchanged which have to move in the opposite direction of the net flow. Thus, a typically dialysis in a batchwise production process is limited by the maximal concentration difference not yet causing a net flow.

Therefore, if it is desired to achieve an efficient exchange of small molecules between the membrane sides the net volume flow across the membranes in the unit for ultrafiltration and purification described herein is as far as possible minimized so that, as in a dialysis method, the transport as far as possible takes place by diffusion. Diffusive transport is in particular achieved when the dilution or concentration of the non-permeating substances takes place with a factor of at most 1 to 5, preferably with a factor of 1 to 2, particularly preferably with a factor of 1.

As used herein the term "capillary ultrafiltration module" refers to an ultrafiltration module comprising capillaries as membrane filters and/or flat sheet membranes as membrane filters.

Thus, a capillary ultrafiltration module can also comprise only capillaries as membrane filters or only flat sheet membranes as membrane filters or both capillaries membrane filters and flat sheet membranes as membrane filters.

For changing the capillary ultrafiltration module, the product stream is usually interrupted and an interim bag fills for the duration of the change.

Through the use of units for sterile filtration for further reduction of the bioburden, the lifetime of the particular modules can be improved such that an interruption and the capture (= storage) of the product stream in an interim bag for the duration of the change is justifiable.

As used herein the term "biological macromolecular product" refers to a large biological molecule commonly created by polymerization of smaller subunits (monomers) such as a biopolymer. Examples include nucleic acids, peptides proteins, carbohydrates, polyphenols, such as protein, commonly created by polymerization of smaller subunits (monomers).

As used herein the term "peptide" refers to a polymer of amino acids of relatively short length (e.g. less than 50 amino acids). The polymer may be linear or branched, it may comprise modified amino acids, and it may be interrupted by non-amino acids. The term also encompasses an amino acid polymer that has been modified; for example, by disulfide bond formation, glycosylation, lipidation, acetylation, phosphorylation, or any other manipulation, such as conjugation with a labeling component, such as but not limited to, fluorescent markers, particles, biotin, beads, proteins, radioactive labels, chemiluminescent tags, bioluminescent labels, and the like.

As used herein the term "protein" refers to a polypeptide of amino acids. The term encompasses proteins that may be full-length, wild-type, or fragments thereof. The protein may be human, non- human, and an artificial or chemical mimetic of a corresponding naturally occurring amino acid, as well as to naturally occurring amino acid polymers and non- naturally occurring amino acid polymer.

Preferably the protein is a therapeutic protein.

As used herein the term "therapeutic protein" refers to a protein that can be administered to an organism to elicit a biological or medical response of a tissue, an organ or a system of said organism.

In a preferred embodiment the biological macromolecular product is a biological i.e. a pharmaceutical drug product manufactured in, extracted from, or semisynthesized from biological sources.

Even more preferably the protein is an antibody.

The term "antibody" as used herein refers to a binding molecule such as an immunoglobulin or immunologically active portion of an immunoglobulin, i.e., a molecule that contains an antigen-binding site.

As used herein the term "small molecule drug" refers to a low molecular weight (<900 daltons) compound that may help regulate a biological process.

As used herein, the term "nucleic acid" refers to deoxyribonucleotides or ribonucleotides and polymers thereof in either single- or double-stranded form. Unless specifically limited, the terms encompass nucleic acids containing analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g. degenerate codon substitutions) and complementary sequences as well as the sequence explicitly indicated.

In a preferred embodiment of the unit for ultrafiltration and purification described herein the guard purifier is a depth filter, which can bind the substance to be separated from the product stream and/or a column which can bind the substance to be separated from the product stream.

An example of a guard purifier is an activated carbon depth filter,

An example of an activated carbon depth filter is a MCR4023CL3 Millistak+® Depth Filter from Merck Millipore.

In a preferred embodiment of the unit for ultrafiltration and purification described herein the unit is a unit for continuous ultrafiltration and continuous purification.

As used herein, the term "continuous" refers to the fact that the input of the product stream into a unit for ultrafiltration and purification and the removal of the product stream from the unit for ultrafiltration and purification place without interruption. In other words, a subsequent unit operation can start processing the product stream before a first unit operation has finished processing the product stream.

As used herein, the term "batchwise" refers to the fact the usually individual production cycles are handled discontinuously in a batchwise manner, with the entire product being removed after completion of a production cycle. To produce again, it is then necessary to start a separate new product cycle/ batch.

The highly regulated pharmaceutical production requires great effort in terms of time, technology and personnel to provide cleaned and sterilized facilities in order to ensure a sterile product. Moreover, to reliably avoid cross-contamination in the event of a product changeover in a multipurpose system or between two product batches a very complex cleaning validation is mandatory, which again requires great effort in terms of time and personnel. Thus, a continuous process for the production of ADCs is advantageous.

In principle, the currently employed method of batchwise diafiltration of the solution containing antibody drug conjugates as well as unbound drugs using high diafiltration factors could be performed in an alternating mode, in order to simulate a continuous process with a batch processing step. In other words, to achieve a continuous product stream one could employ two batch-diafiltrations in an alternating fashion, i.e. while diafiltration is carried out in a first diafiltration unit the second unit is prepared for diafiltration via adjusting the parameters such as buffer concentration, ADC-concentration and flow rate. After a specific predetermined time the processing is switched from the first diafiltration unit to the second unit, and the first diafiltration unit is regenerated and prepared again for diafiltration via adjusting the necessary parameters. Thus, a constant product stream could be realized.

However, using this method could negatively influence the product quality since high wash rates cause longer processing times, a higher input stirring energy and higher pump rates and thus ultimately to a higher degree of shear forces. Hence, all of these factors can potentially damage the fragile ADC complexes resulting in the risk of formation of subvisible particles and aggregate formation upon long-time storage.

In contrast, the described unit for ultrafiltration and purification allows an operation mode of the ultrafiltration module that differs from typical ultrafiltration modules, in that
a) there is no product solution, which is placed in a defined container
b) the product stream is not pumped in a circuit via the ultrafiltration membrane

In contrast, the described unit allows a truly continuous operation mode since the product stream continuously enters the unit, the washing fluid is continuous removed and each part of the product stream and the washing fluid, respectively only passes the capillary ultrafiltration module one time.

Moreover, the described unit for ultrafiltration and purification allows an operation mode of the ultrafiltration module that differs from typical dialysis, in that at least one positive displacement pump conveys the product stream from the capillaries. This at least one positive displacement pump generates a positive or negative pressure, which is not present in a typical dialysis, since a typical dialysis relies exclusively on diffusion. Hence, the positive displacement pump ensures that the rate with which the product stream enters the unit for ultrafiltration and purification resembles or is equal to the rate with which the retentate leaves the unit for ultrafiltration and purification. This set-up therefore minimizes undesired net flows from capillary interior, i.e. from one side of the filtration membrane, to the capillary exterior, i.e. to the one side of the filtration membrane, or vice versa. Due to this regulation the concentrations of molecules to be exchanged via the filtration membrane can differ to great extent without the occurrence of undesired net-flows, since the flow from capillary interior to the capillary exterior is not solely due to diffusion as it would be the case in a dialysis.

In a further preferred embodiment of the unit for ultrafiltration and purification the biological macromolecular product is an ADC.

In a further preferred embodiment of the unit for ultrafiltration and purification the substance to be separated from the product stream is a toxophore

In a further preferred embodiment of the unit for ultrafiltration and purification the unit for ultrafiltration and purification described herein is disposable and/or for single use.

Maintenance of sterility is a challenge for a (semi)continuous production of biopharmaceutical and biological macromolecular products. Thus, preferably in the unit for ultrafiltration and purification described, all components are connected together by tubes, in particular disposable tubes. For example, biocompatible tubes Pharmed BPT® (heat-resistant silicone tubes) are used. The other components of the plant are also preferably disposable components; in particular, components selected from the group of disposable filtration elements, disposable valves, disposable sensors (flow, pH, conductivity, UV, pressure), disposable tubes, disposable membranes, disposable connectors, disposable containers and disposable sampling systems are used. Means for conveying liquid, in particular pumps, are also preferably disposable pumps.

Preferred washing fluids such as formulation buffers e.g. aqueous solution comprising sodium chloride, Tris, etc. at a pH of about 6,8-7,5. are known in the art.

In a further preferred embodiment of the unit for ultrafiltration and purification the substance to be separated from the product stream is a toxophore and said toxophore is cleared with a rate as determined according to the guidelines of the International Council for Harmonisation of Technical Requirements for Pharmaceuticals for Human Use (ICH), which provide the recommended daily dose for a given toxophore.

In another aspect the current invention relates to a method for continuous ultrafiltration and continuous purification of a product stream containing biological macromolecular product as well as a substance to be separated from the product stream, characterized in that
- the product stream is washed with a washing fluid via at least one capillary ultrafiltration membrane of a capillary ultrafiltration module,
- the product stream is conveyed into the capillary and the washing fluid is conveyed over the outside of the capillary,
- the product stream and the washing fluid are continuously fed into the capillary ultrafiltration module, and are continuously removed from the capillary ultrafiltration module,
- the product stream and the washing fluid are not circulated into the capillary ultrafiltration module
- the removal of the product stream is regulated such that no undesired net flows can pass from the capillary interior to the capillary exterior or vice versa
- all fluid leaving the at least one capillary ultrafiltration module in the direction of the product stream is passed through at least one guard purifier.

The feature that "the product stream and the washing fluid are not circulated into the capillary ultrafiltration module" is an immanent feature of resulting from the design and set-up of the method for continuous ultrafiltration and continuous purification, since every part of the product stream only passes the capillary ultrafiltration membrane of a capillary ultrafiltration module one time.

The feature that the "removal of the product stream is regulated such that no undesired net flows can pass from the capillary interior to the capillary exterior or vice versa (i.e. from the one side of the capillary filtration membrane to the other side of the capillary filtration membrane) " is achieved via providing at least one positive displacement pump such as a peristaltic pump that conveys the product stream from the capillaries. Alternatively a control valve and/or a flow control can be used to ensure that the removal of the product stream is regulated such that no undesired net flows can pass from the capillary interior to the capillary exterior or vice versa.

In a further aspect, the invention relates to a production plant which comprises at least one unit for continuous ultrafiltration and purification as described herein.

Preferably, said production plant is a production plant for continuous or semi-continuous implementation of one or more production steps in the production of a biological macromolecular product.

As a consequence of the fact that the unit for ultrafiltration and purification described herein allows for a truly continuous ultrafiltration and purification said unit also enables the continuous production of antibody drug conjugates in a production process where a part of the product stream continuously enters and another part of the product stream continuously leaves a specific unit operation such as the unit for ultrafiltration and purification as described herein. In other words, a subsequent unit operation starts processing the product stream before the first unit operation has finished processing the product stream.

However, as described above it is also possible to use the unit for ultrafiltration and purification in a semi-continuous or a batch process. If in such a setting for example the product is an antibody-drug conjugate and a toxophore is to be removed from the product stream, a holding tank can precede the unit for ultrafiltration and purification described herein and the retentate may be collected in another holding tank.

Said production steps can e.g. be selected from the group of: cell culture, cell retention, chromatography, viral inactivation.

Preferably, in said production plant, all components are connected together by tubes, in particular disposable tubes. For example, biocompatible tubes Pharmed BPT® (heat-resistant silicone tubes) are used. The other components of the plant are also preferably disposable components; in particular, components selected from the group of disposable reactors, disposable filtration elements, disposable valves, disposable sensors (flow, pH, conductivity, UV, pressure), disposable cell retention systems, disposable tubes, disposable membranes, disposable connectors, disposable chromatography columns, disposable containers and disposable sampling systems are used. Means for conveying liquid, in particular pumps, are also preferably disposable pumps.

In another aspect the invention relates to a method for the continuous, germ-reduced production and/or processing of an antibody-drug-conjugate using at least one continuous ultrafiltration and continuous purification (diafiltration) of the product stream as described herein,
wherein the process is carried out in a closed and modular manner.

As used herein the term "closed" means that the method described is operated in such a way that the fluid stream is not exposed to the room environment. Materials, objects, buffers, and the like can be added from outside, wherein, however, this addition takes place in such a way that exposure of the fluid stream to the room environment is avoided. Thus, sterile filters may be used to provide effective barriers from contaminants in the environment. Examples of closed systems include sterile single use bags supplied with integrated aseptic connection devices. Moreover, process systems may be opened but are "rendered closed" by a cleaning, sanitization and/or sterilization process that is appropriate or consistent with the process requirements, whether sterile, aseptic or low bioburden. Examples include process vessels that may be CIP'd and SIP'd between uses. Non-sterile systems such as chromatography or some filtration systems may also be rendered closed in low bioburden operations if appropriate measures are taken during the particular system setup. As used herein the term "closed" refers to both "functionally closed" as well as "closed" systems and as stated above generally refers to the fact that a described method is operated in such a way that the fluid stream is not exposed to the room environment.

As used herein the term "pathogen-reduced" is used interchangeable with "microbe-reduced" and "germ-reduced" and refers to a state of reduced pathogenic count, e.g. reduced viral count, i.e. a pathogenic count per area or volume unit of close to zero that is achievable by means of a suitable germ-reducing method, wherein this germ-reducing method can be selected from gamma irradiation, beta irradiation, autoclaving, Ethylene Oxide (ETO) treatment, Ozone treatment, "Steam-In-Place" (SIP) and/or Heat in Place treatment or treatment with sanitization agent like 1 M NaOH.

In order to provide a biological macromolecular product - in this example an a product stream is subjected to an ultrafiltration and purification as described herein, i.e. the product stream is washed with a washing fluid via at least one capillary ultrafiltration membrane of a capillary ultrafiltration module, wherein the product stream is conveyed into the capillary and the washing fluid is conveyed over the outside of the capillary and the product stream and the washing fluid are continuously fed into the capillary ultrafiltration module, and are continuously removed from the capillary ultrafiltration module and the product stream and the washing fluid are not circulated into the capillary ultrafiltration module and the removal of the product stream is regulated such that no undesired net flows can pass from the capillary interior to the capillary exterior or vice versa and all fluid leaving the at least one capillary ultrafiltration module in the direction of the product stream is passed through at least one guard purifier. For conveying the product stream (= feed stream) into the capillaries and the washing fluid (= permeate) on the outsides of the capillaries of the ultrafiltration module in each case one pump is used. For the controlled removal of the product stream from the capillaries (= retentates), one further pump is used. Through the use of this pump (= retentate pump), control of the removal of the retentate is simplified. This is advantageous as there are no adequate flow sensors which reliably measure the low flow rates (≤ 100 ml/min) usually employed in this continuous process. Particularly preferably, peristaltic pumps can be used here with the advantage that peristaltic disposable pumps are commercially available, so that sterility and also disposable technology are available. Moreover, a pump is used for the controlled removal of the wash fluid (permeate).

### Figures

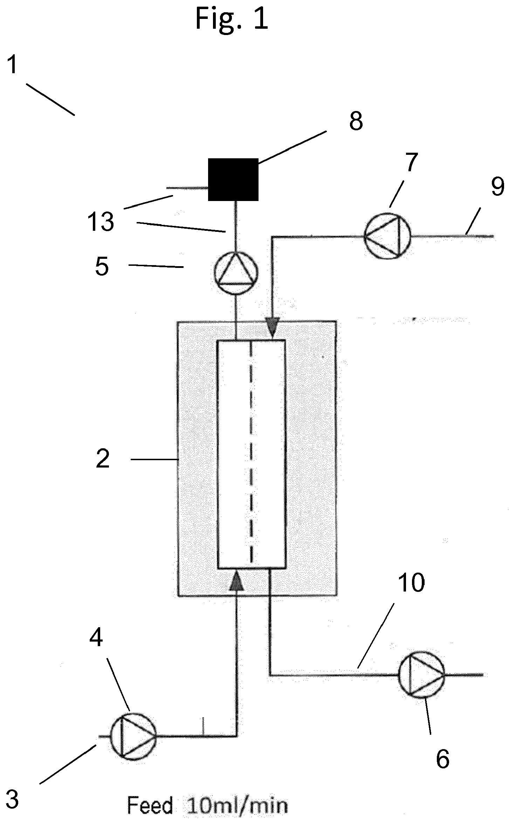

FIG. 1 shows a schematic drawing of a unit for ultrafiltration and purification (1) as described herein. In detail said unit for ultrafiltration and purification (1) comprises one capillary ultrafiltration module (2) - here a Gambro Polyflux® 2H with capillaries that allow molecules from 1-10.0000 Da to pass one pump (4) that conveys the product stream (3) - in this example the product stream comprises antibody drug conjugates as well as unbound toxophores - into the capillaries, one positive displacement pump (5) that conveys the product stream from the capillaries of the ultrafiltration module (2), two pumps (6,7) that pass a washing fluid - in this case a diafiltration buffer (9) - over the outside of the capillaries and one activated carbon depth filter (8), through which all fluid leaving the at least one capillary ultrafiltration module in the direction of the product stream - i.e. the retentate (13) - is passed. The term retentate refers to both the product stream leaving the unit for ultrafiltration and purification and the product stream leaving the activated carbon depth filter. The diafiltration buffer leaving the capillary ultrafiltration module (2) contains the unbound toxophores, i.e. constitutes the permeate (10) in this example. In this specific setting, the product stream is entering the unit for ultrafiltration and purification (1) at a rate of 10 ml/min. This is also the rate at which the product stream passes the active carbon depth filter (8). The diafiltration buffer (9) enters and leaves the capillary ultrafiltration module (2) at a rate of 30 ml/min. Thus, the driving forces that cause the separation of the product stream and the toxophores in this case are the particle size allowed to pass by the filtration membranes in the capillaries of the capillary ultrafiltration module, the difference in flow rate between the product stream and the diafiltration buffer that cause a pressure, as well as the differences in gradient e.g. of the between the product stream and the diafiltration buffer.

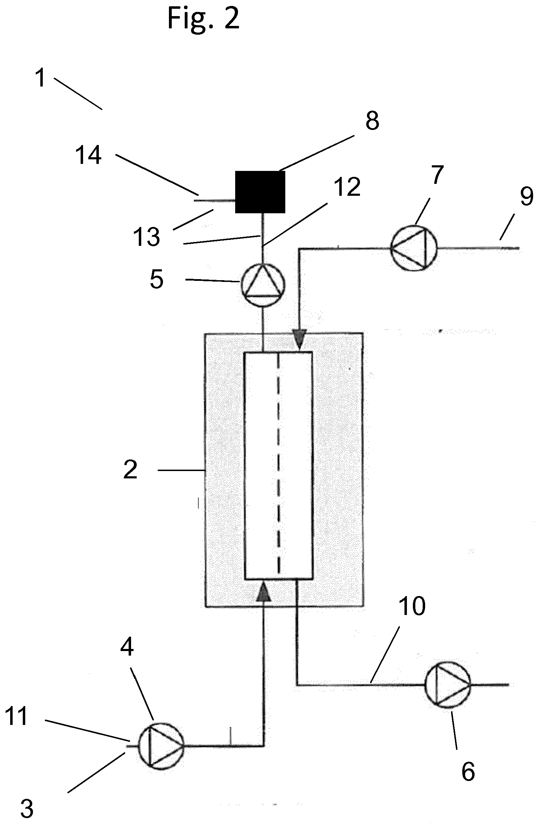

FIG. 2 corresponds to FIG. 1, but in addition to the details of FIG. 1 a point is depicted where a
   sample (11) is taken before the product stream enters the unit for ultrafiltration and purification. A second sample (12) is taken before the retentate (13) enters the activated carbon depth filter (8) and a final sample (14) is taken after the retentate (13) has passed the activated carbon depth filter (8).

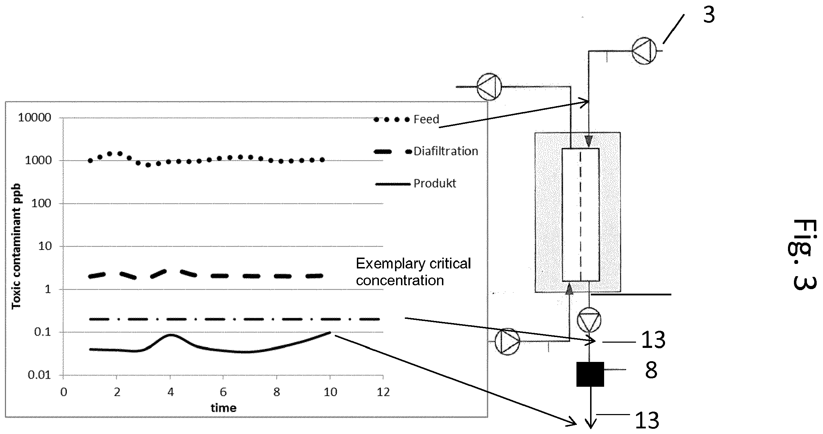

FIG. 3 Shows a schematic diagram of the toxophore concentration in parts per billion (ppb) before the product stream (feed) (3) enters the unit for ultrafiltration and purification, before the product stream (retentate) (13) enters the activated carbon depth filter (8) and after the product stream (13) leaves the activated carbon depth filter. It is depicted that the toxophore concentration of the product stream, which is already below the critical concentration, before the product stream enters the activated carbon depth filter is further reduced by the activated carbon depth filter.

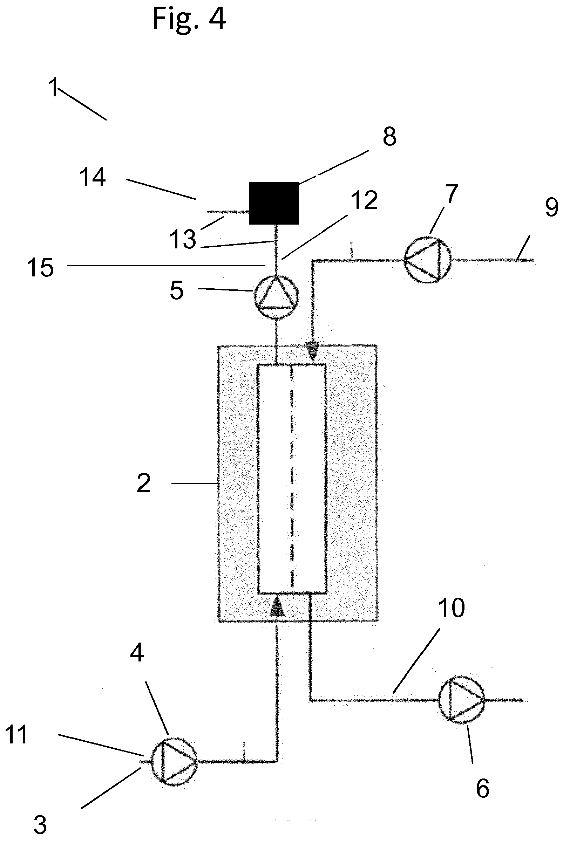

FIG. 4 The set-up is as described above for FIG 2, but the product stream was spiked with an additional load of toxophore (15) prior to entering the active carbon depth filter in order to demonstrate that the activated carbon depth filter can act as safeguard to ensure that the concentration of the toxophore is below the critical threshold once the product stream leaves the unit for ultrafiltration and purification.

### Examples

In order to demonstrate that the unit for ultrafiltration and purification as described herein reliably and effectively separates toxophores from a product stream comprising toxophores and antibody-toxophore conjugates the following experiments are carried out. A unit for ultrafiltration and purification as depicted in and described with reference to Fig. 2 is operated with a flow rate of the product stream of 5ml/min and a flow rate of the wash buffer of 15 ml/min. A sample (11) is taken before the product stream enters the unit for ultrafiltration and purification. A second sample (12) is taken before the retentate enters the activated carbon depth filter (8) and a final sample (14) is taken after the retentate has passed the activated carbon depth filter (8). As shown in FIG 3 the toxophore concentration is above the critical threshold in the sample (11) taken before the product stream enters the unit for ultrafiltration and purification. As shown in FIG 3 the toxophore concentration is below the critical threshold in the sample (14) taken after the product stream has passed the activated carbon depth filter.

## Claims

1. Unit for ultrafiltration and purification (1) of a product stream (3) containing a biological macromolecular product as well as a substance to be separated from the product stream, comprising at least one capillary ultrafiltration module (2), **characterized in that**
- at least one pump (4) conveys the product stream into the capillaries of the at least one capillary ultrafiltration module,
- at least one positive displacement pump (5) conveys the product stream from the capillaries and
- at least one further pump (6) passes a washing fluid (9) over the outside of the capillaries,
- the unit includes no measure for circulating the product stream and the washing fluid into the ultrafiltration module, as well as
at least one guard purifier (8), through which all fluid leaving the at least one capillary ultrafiltration module in the direction of the product stream is passed.

2. The unit for ultrafiltration and purification according to claim 1, wherein the guard purifier is a depth filter which can bind the substance to be separated from the product stream and/or a column which can bind the substance to be separated from the product stream.

3. The unit for ultrafiltration and purification according to claims 1-2, wherein the guard purifier is an activated carbon depth filter.

4. The unit for ultrafiltration and purification according to according to anyone of the preceding claims, wherein the unit is a unit for continuous ultrafiltration and continuous purification.

5. The unit for ultrafiltration and purification according to according to anyone of the preceding claims, wherein the biological macromolecular product is an antibody-drug-conjugate.

6. The unit for ultrafiltration and purification according to anyone of the preceding claims, wherein substance to be separated from the product stream is a toxophore.

7. The unit for ultrafiltration and purification according to anyone of the preceding claims, wherein the unit is disposable and/or for single use.

8. Method for continuous ultrafiltration and continuous purification of a product stream containing biological macromolecular product as well as a substance to be separated from the product stream, **characterized in that**
- the product stream is washed with a washing fluid via at least one capillary ultrafiltration membrane of a capillary ultrafiltration module,
- the product stream is conveyed into the capillary and the washing fluid is conveyed over the outside of the capillary,
- the product stream and the washing fluid are continuously fed into the capillary ultrafiltration module, and are continuously removed from the capillary ultrafiltration module,
- the product stream and the washing fluid are not circulated into the capillary ultrafiltration module
- the removal of the product stream is regulated such that no undesired net flows can pass from the capillary interior to the capillary exterior or vice versa
- all fluid leaving the at least one capillary ultrafiltration module in the direction of the product stream is passed through at least one guard purifier.

9. Use of the method according to claim 8 in a process for the continuous, germ-reduced production and/or processing of an antibody-drug-conjugate, wherein the process is carried out in a closed and modular manner.
